# EUROPEAN PATENT APPLICATION

(11) **EP 4 382 080 A1**
(43) Date of publication of application: **12.06.2024**
(21) Application number: 21952193.7
(22) Date of filing: 03.08.2021
(51) Int. Cl.: A61F 2/24

(54) **HEART VALVE CLAMP AND MANUFACTURING METHOD THEREFOR**

(71) Applicant: Shanghai Shape Memory Alloy Co., Ltd., Shanghai, 201612 (CN)
(72) Inventor: ZHAO, Yang, Shanghai 201612 (CN); LIU, Xiaojian, Shanghai 201612 (CN); ZHANG, Yuxin, Shanghai 201612 (CN)
(74) Representative: Lavoix
(86) International application number: PCT/CN2021/110347
(87) International publication number: WO 2023/010284

(57) **Abstract**

A heart valve clamp and a manufacturing method therefor. The heart valve clamp includes a fixed arm mechanism (10) and a clamping arm mechanism (20). The clamping arm mechanism (20) includes a clamping arm base (21), a clamping arm (22) and a first supporting member (23). The width of the clamping arm (22) is a first width. The first supporting member (23) is connected to a distal end face of the clamping arm (22) and extends distally in the extending direction of the clamping arm (22). The width of the first supporting member (23) is a second width, and the second width is greater than the first width. Comparing the heart valve clamp with an existing heart valve clamp, only the first supporting member which has a width greater than that of the clamping arm is provided on the distal end face of the clamping arm, only the width of an operating part of the clamp is increased, and the overall volume of a conveying system and the clamp are not required to be increased, that is, a clamping area of a valve leaflet is effectively increased, the contact area between the clamp and the valve leaflet after the clamp clamps the valve leaflet is increased, a clamping force of the clamp on the valve leaflet is uniform, the valve leaflet is prevented from being damaged, and the difficulty and risks of the operation are reduced.

## Description

### TECHNICAL FIELD

The present application relates to the technical field of medical devices, and specifically to a heart valve clamp and a manufacturing method therefor.

### BACKGROUND

Mitral valve is a one-way valve located between the left atrium and the left ventricle of the heart. A normal and healthy mitral valve can control the blood to flow from the left atrium to the left ventricle and prevent the blood from flowing from the left ventricle to the left atrium. With the development of the society and the aging of the population, the incidence of mitral regurgitation (MR) exhibits a significantly increasing trend, and it has become a common heart valve disease now.

The current treatment of mitral regurgitation has undergone the era of traditional surgical median sternotomy and the recent minimally invasive small incision surgery and is ushering in an era of catheter interventional therapy. Interventional minimally invasive techniques for the treatment of mitral regurgitation have become one of the few hottest research directions in the interventional cardiology, and tremendous progress has been made in the interventional treatment methods for MR with less trauma, fewer complications, and lower cost.

The interventional therapy techniques for mitral regurgitation may be divided into two classes: one is transcatheter mitral valve repair (TMVR), such as MitraClip, PASCAL, ValveClam, Cardioband, Mitralign, NeoChord, etc.; the other is transcatheter mitral valve implantation (TMVI). The repair surgery is currently the main treatment method, but there are still some problems with the current technique. For example, the MitralClip is designed to achieve the treatment purpose by clamping the anterior and posterior valve leaflets of the mitral valve and turning a large single orifice into two small orifices, but the problem is that there is a long path to get to the implantation position and the operation is too complicated, so this technique needs to be improved.

The heart valve clamp in the existing technology includes a clamping arm mechanism and a fixed arm mechanism. The clamping arm mechanism includes a clamping arm and a traction arm, and the fixed arm mechanism includes a fixed arm. The clamping arm mechanism is fixedly connected to the fixed arm mechanism. The conveying system includes a conveying sheath tube and a traction line. During the surgery, the conveying sheath tube is connected to the bottom part of the clamping arm mechanism, the fixed arm is connected to the traction line. The heart valve clamp is conveyed to the location of the left atrium first through the conveying system, and then pushed to the lower part of the valve. The conveying sheath tube was pulled downward to expand the clamping arm to a certain angle, and the clamp is pushed upward so that the clamping arm holds the valve leaflet from the side of the ventricle. The traction line is then released to cause the fixed arm to expand and press the valve leaflet firmly against the clamping arm. After confirming that the clamping location of the valve leaflet is appropriate and the effect of regurgitation treatment is achieved, the clamping arm is folded and closed to complete the clamping steps of the heart valve clamp.

For the above heart valve clamp, the clamping arm mechanism has a tube structure, the clamping arm is formed by cutting the tube structure, and the clamping arm has a relatively narrow width. Therefore, when the clamping arm clamps a valve leaflet, the contact area between the clamping arm and the valve leaflet is small, and it is easy to cause uneven clamping force due to stress concentration, thus damaging the valve leaflet. The clamping arm mechanism is provided with a hanging head at the bottom, through which it is connected to the conveying system. The tube diameter of the clamping arm mechanism matches the diameter of the conveying system. If a tube with a slightly larger diameter is directly used to cut the clamping arm to obtain a wider clamping arm, the overall size of the conveying system and the clamp will be increased, thereby increasing the difficulty of the surgery and affecting the adaptability of human after the implantation of the clamp.

### SUMMARY OF THE INVENTION

Therefore, the technical problem to be solved in the present application is to overcome the defects of the heart valve clamp in the existing technology, of which the clamping arm has a relatively narrow width, and it is easy to damage the valve leaflets during the clamping process.

According to an aspect of the present application, a heart valve clamp is provided, which includes:
a fixed arm mechanism, the fixed arm mechanism includes a fixed arm;
a clamping arm mechanism, the clamping arm mechanism includes a clamping arm base, a clamping arm and a first supporting member, where the clamping arm base is connected to the fixed arm mechanism, the clamping arm extends distally from the clamping arm base; a width of the clamping arm is a first width;
where the first supporting member is connected to a distal end face of the clamping arm and extends distally in the extending direction of the clamping arm, a width of the first supporting member is a second width, and the second width is greater than the first width.

Optionally, for the heart valve clamp, the first supporting member is hollow in its central part to form a shape of a closed ring.

Optionally, for the heart valve clamp, before shaping, an initial length of the first supporting member in the extending direction of the clamping arm is greater than an initial width of the first supporting member.

Optionally, for the heart valve clamp, the first supporting member is oval-shaped.

Optionally, for the heart valve clamp, the fixed arm mechanism is arranged inside the clamping arm mechanism, and an outer wall surface of the fixed arm is an arc-shaped surface;
a bottom part of the first supporting member connected to the clamping arm and a top part thereof opposite to the bottom part are both inclined outward, and the central part of the first supporting member is concave inward; and when the clamp is in a folded state, the top part of the first supporting member wraps around and fits over a distal end face of the fixed arm, and a central part of the fixed arm protrudes into a hollow area of the first supporting member.

Optionally, for the heart valve clamp, a bottom part of the first supporting member is provided with a first connecting member extending toward the clamping arm.

Optionally, for the heart valve clamp, the first supporting member is integrally formed with the clamping arm.

Optionally, for the heart valve clamp, the first supporting member is provided with a second supporting member extending in the extending direction of the clamping arm.

According to another aspect of the present application, a method for manufacturing the heart valve clamp as described in any one of above is provided, the method includes:
processing to obtain an initial first supporting member and a clamping arm; where a width of the clamping arm is a first width;
shaping the initial first supporting member to obtain a first supporting member having a width which is a second width, and connecting the first supporting member to a distal end face of the clamping arm; where the second width is greater than the first width.

Optionally, for the above method for manufacturing the heart valve clamp,
in the step of processing to obtain the initial first supporting member and the clamping arm, the clamping arm and the initial first supporting member with a hollow central part that has a shape of a closed ring are formed by cutting a tubular structure; and the initial length of the first supporting member in the extending direction of the clamping arm is greater than the initial width of the first supporting member;
in the step of shaping the initial first supporting member, a mold is placed in a hollow area of the initial first supporting member to expand the initial first supporting member for heat shaping.

The technical solutions of the present application have the following advantages:
1. During a surgery with the heart valve clamp provided in the present application, the conveying system is connected to the heart valve clamp. The heart valve clamp is conveyed to the location of the left atrium first through the conveying system, and then pushed to the lower part of the valve. The clamping arm is expanded by moving the conveying sheath tube, and the first supporting member is driven to expand synchronously. When both the clamping arm and the first supporting member are expanded to form a certain angle, the heart valve clamp is pushed upward so that the clamping arm and the first supporting member hold the valve leaflet from the bottom part of the valve. The fixed arm is then released, and the fixing member presses from the top part of the valve against on the clamping arm and the first supporting arm. After confirming that the clamping position of the valve leaflet is appropriate and the effect of regurgitation treatment is achieved, the conveying sheath tube is folded, and the lower clamping arm is folded and closed to complete the clamping steps of the heart valve clamp.
   Compared with an existing heart valve clamp, only a first supporting member which has a width greater than that of the clamping arm is provided on a distal end face of the clamping arm, only the width of an operating part of the clamp is increased, and the overall volume of a conveying system and the clamp are not required to be increased, that is, a clamping area of a valve leaflet is effectively increased, the contact area between the clamp and the valve leaflet after the clamp clamps the valve leaflet is increased, a clamping force of the clamp on the valve leaflet is uniform, the valve leaflet is prevented from being damaged, and the difficulty and risks of the operation are greatly reduced.
2. In the method for manufacturing a heart valve clamp as described in the present application, a first supporting member and a clamping arm are first processed, the first supporting member is then shaped to obtain a first supporting member having a width greater than that of the clamping arm, which can effectively increase the clamping area of the clamping arm and the first supporting member when they clamp the valve leaflet. The clamping force of the clamp on the valve leaflet is uniform, which prevents the valve leaflet from being damaged, thus greatly reducing the operation risk during the implantation of the clamp. Moreover, after the first supporting member is processed, it can be shaped according to the requirement of valves with different structures to obtain first supporting members of different widths.

### BRIEF DESCRIPTION OF THE DRAWINGS

To describe the technical solutions in the embodiments of the present application or in the prior art more clearly, drawings for use in the description of the embodiments or the prior art will be introduced briefly below. Obviously, the drawings described below are merely some implementations of the present application, and for those of ordinary skill in the art, other drawings may also be obtained based on these drawings without creative work.
Fig. 1 is a schematic diagram of a heart valve clamp provided in Embodiment 1 of the present application when it is folded;
Fig. 2 is another schematic diagram of a heart valve clamp provided in Embodiment 1 of the present application when it is folded;
Fig. 3 is a schematic diagram of a clamping arm mechanism in Fig. 1;
Fig. 4 is a front view of the clamping arm mechanism provided in Embodiment 1 of the present application;
Fig. 5 is an expended view of the clamping arm mechanism provided in Embodiment 1 of the present application;
Fig. 6 is a schematic diagram of a fixed arm mechanism in Fig. 1;
Fig. 7 is a front view of the fixed arm mechanism provided in Embodiment 1 of the present application;
Fig. 8 is an expended view of the fixed arm mechanism provided in Embodiment 1 of the present application;
Fig. 9 is a schematic diagram of a fixed arm of the fixed arm mechanism provided in Embodiment 1 of the present application when it is expanded;
Fig. 10 is a schematic diagram of the heart valve clamp provided in Embodiment 1 of the present application when it is expanded;
Fig. 11 is a schematic diagram of the heart valve clamp provided in Embodiment 1 of the present application when it is implanted into the body;
Fig. 12 is a schematic diagram of a heart valve clamp provided in a variant embodiment of the present application when it is folded;
Fig. 13 is a schematic diagram of the clamping arm mechanism in Fig. 11;
Fig. 14 is a front view of the clamping arm mechanism provided in a variant embodiment of the present application.

### Reference numerals:

10-fixed arm mechanism; 11-fixed arm; 12-fixed arm base; 13-barb; 14-traction hole; 20-clamping arm mechanism; 21-clamping arm base; 22-clamping arm; 23-first supporting member; 231-first connecting member; 24-second supporting member; 25-traction arm; 26-traction arm base; 27-hanging head; 30-conveying sheath tube; 40-push rod.

### DETAILED DESCRIPTION

Technical solutions of the present application will be described below clearly and completely in conjunction with the accompanying drawings. Obviously, the described embodiments are part of, instead of all of embodiments of the present application. All other embodiments obtained by those of ordinary skill in the art without creative work, based on the embodiments in the present application, fall into the protection scope of the present application.

In description of the present application, it is to be noted that orientation or location relations denoted by the terms "center", "upper", "lower", "left", "right", "vertical", "horizontal", "inner", "outer", etc. are orientation or location relations based on illustration in the drawings, are only intended to facilitate describing the present application and simplify the description, instead of indicating or implying the denoted devices or elements necessarily have specific orientations and are constructed and operated in specific orientations, and thus they should not be construed as limiting the present application. In addition, the terms "first", "second", "third", etc. are only used for description and should not be construed as indicating or implying relative importance.

In the description of the present application, it is to be noted that, unless otherwise expressly specified and defined, the terms "install", "be connected with", and "be connected" should be construed in a broad sense. For example, it may indicate fixing connection, or detachable connection, or integrated connection; it may indicate mechanical connection, or electrical connection; it may indicate direct connection, or indirect connection through an intermediate medium, or internal communication between two elements. For those of ordinary skill in the art, specific meanings of the above-mentioned terms in the present application may be construed according to specific circumstances.

In addition, technical features involved in different implementations of the present application described below may be combined with each other so long as they do not conflict with each other.

### Embodiment 1

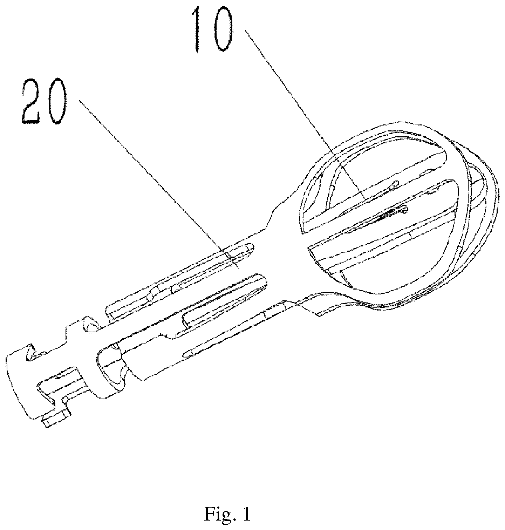

A heart valve clamp is provided in this embodiment, as shown in Figs. 1 to 5, which includes a fixed arm mechanism 10 and a clamping arm mechanism 20. Where, the fixed arm mechanism 10 includes a fixed arm 11, the clamping arm mechanism 20 includes a clamping arm base 21, a clamping arm 22 and a first supporting member 23, the clamping arm base 21 is connected to the fixed arm mechanism 10, the clamping arm 22 extends distally from the clamping arm base 21; when the heart valve clamp is in a folded state, the extending direction of the clamping arm 22 is the length direction, and the direction perpendicular to the extending direction of the clamping arm 22 in the same plane is the width direction; the width of the clamping arm 22 is the first width; the first supporting member 23 is connected to a distal end face of the clamping arm 22 and extends distally in the extending direction of the clamping arm 22, the width of the first supporting member 23 is the second width, and the second width is greater than the first width.

During a surgery with a heart valve clamp of such a structure, the conveying system is connected to the heart valve clamp. The heart valve clamp is conveyed to the location of the left atrium first through the conveying system, and then pushed to the lower part of the valve. The clamping arm 22 is expanded by moving the conveying sheath tube 30, and the first supporting member 23 is driven to expand synchronously. When both the clamping arm 22 and the first supporting member 23 are expanded to form a certain angle, the heart valve clamp is pushed upward so that the clamping arm 22 and the first supporting member 23 hold the valve leaflet from the bottom part of the valve. The fixed arm 11 is then released, and the fixing member presses from the top part of the valve against on the clamping arm 22 and the first supporting arm. After confirming that the clamping position of the valve leaflet is appropriate and the effect of regurgitation treatment is achieved, the conveying sheath tube 30 is folded, and the lower clamping arm 22 is folded and closed to complete the clamping steps of the heart valve clamp.

Compared with the existing heart valve clamp, only a first supporting member 23 which has a width greater than that of the clamping arm 22 is provided on the distal end face of the clamping arm 22, only the width of an operating part of the clamp is increased, and the overall volume of a conveying system and the clamp are not required to be increased, that is, a clamping area of a valve leaflet is effectively increased, the contact area between the clamp and the valve leaflet after the clamp clamps the valve leaflet is increased, a clamping force of the clamp on the valve leaflet is uniform, the valve leaflet is prevented from being damaged, and the difficulty and risks of the operation are greatly reduced.

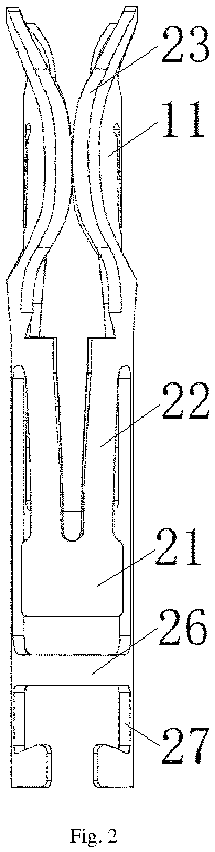

With reference to Figs. 2 to 5, the first supporting member 23 is hollow in its central part to form a shape of a closed ring. For example, the second width H of the first supporting member 23 may be adjusted by means of shaping. The first supporting member 23 is hollow in its central part, which facilitates placing the mold into the first supporting member 23 for the shaping of the first supporting member 23. The width of the first supporting member 23 can be adjusted according to the actual desired valve structure.

Most preferably, before shaping, the initial length of the first supporting member 23 in the extending direction of the clamping arm 22 is greater than the initial width of the first supporting member 23. For example, the first supporting member 23 is oval-shaped with rounded edges and corners, which may prevent the valve leaflet from being damaged when clamping. The initial length of the first supporting member 23 is smaller than its initial width, which facilitates expanding the first supporting member 23 distally in the direction of its width while shaping. When expanded, the length of the initial first supporting member 23 becomes smaller, and its width becomes larger.

With reference to Figs. 1 and 2, the clamping arm mechanism 20 and the fixed arm mechanism 10 are both tubular structure, and the fixed arm mechanism 10 is arranged inside the clamping arm mechanism 20, and the distal peripheral wall of the fixed arm 11 is an arc-shaped surface; the bottom part of the first supporting member 23 connected to the clamping arm 22 and the top part thereof opposite to the bottom part are both inclined outward, and the central part of the first supporting member 23 is concave inward. That is, with reference to Fig. 2, the top part and bottom part of the first supporting member 23 are both inclined outward and the inner wall is an arc-shaped surface, and the arc-shaped surfaces of the top part and bottom part of the first supporting member 23 are matched with the arc-shaped surface of the distal peripheral wall of the fixed arm 11. When the clamp is in a folded state, the top part of the first supporting member 23 wraps around and fits over the distal end face of the fixed arm 11. When the valve leaflet is clamped, the first supporting member 23 can effectively mesh with the arc-shaped surface of the fixed arm 11, causing stable clamping and small damage on the valve leaflet. Most preferably, with reference to Fig. 2, the central part of the first supporting member 23 is concave inward and there is a straight transition section in the central part. When the clamp is in a folded state, the inner wall of the straight transition sections in the central parts of the two first supporting member 23 fit close to each other, so that the central part of the fixed arm 11 protrudes into a hollow area of the first supporting member 23. When the valve leaflet is clamped, the straight transition section and the central part of the fixed arm 11 can clamp over the valve leaflet firmly to keep a stable clamping.

The first supporting member 23 is provided with a first connecting member 231 extending toward the clamping arm 22 on its bottom, to increase the connection strength between the first supporting member 23 and the clamping arm 22, the strength of the first supporting member 23, and the clamping strength after clamping the valve leaflet. Most preferably, the clamping arm mechanism 20 is formed by cutting a whole tube, the first supporting member 23 is integrally formed with the clamping arm mechanism 20, so the clamping arm mechanism 20 has good integrity and is easy to process. In addition, there is a firm connection between various components, resulting in a stable clamping force after clamping the valve leaflet.

Most preferably, with reference to Fig. 1 and Figs. 3 to 5, the first supporting member 23 is provided with a second supporting member 24 extending in the extending direction of the clamping arm 22. For example, the second supporting member 24, in an elongated shape, is welded to an inner wall surface of the first supporting member 23 and is located at the middle part of the first supporting member 23 in its width direction, so as to increase the strength and supporting force of the first supporting member 23, thereby further enhancing the stability after clamping the valve leaflet.

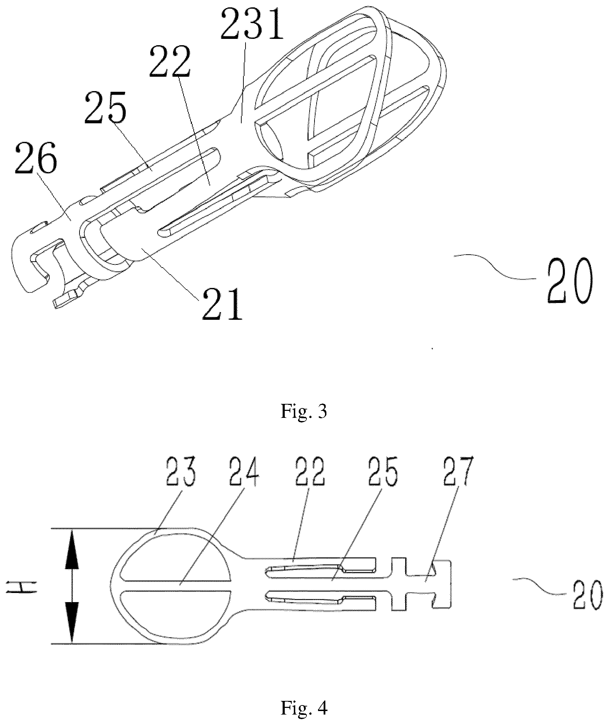

With reference to Figs. 3 to 5, the two clamping arms 22 are disposed symmetrically. The clamping arm mechanism 20 also includes a traction arm base 26, two symmetrically disposed traction arms 25 and a hanging head 27. The traction arm base 26 is located at the bottom part of the clamping arm base 21, and the traction arm base 26 is in a hollow cylindrical shape. The traction arm 25 extends distally from the traction arm base 26 to converge with the clamping arm 22, and the converging section is connected with the first connecting member 231 of the first supporting member 23. A hanging head 27 is provided at the bottom part of the traction arm base 26, and the hanging head 27 is a hook in an inverted "T" shape. The conveying sheath tube 30 is provided with a T-shaped groove that matches with the hanging head 27. During the surgery, the clamp is connected to the T-shaped groove of the conveying sheath tube 30 in a matching manner through the hanging head 27.

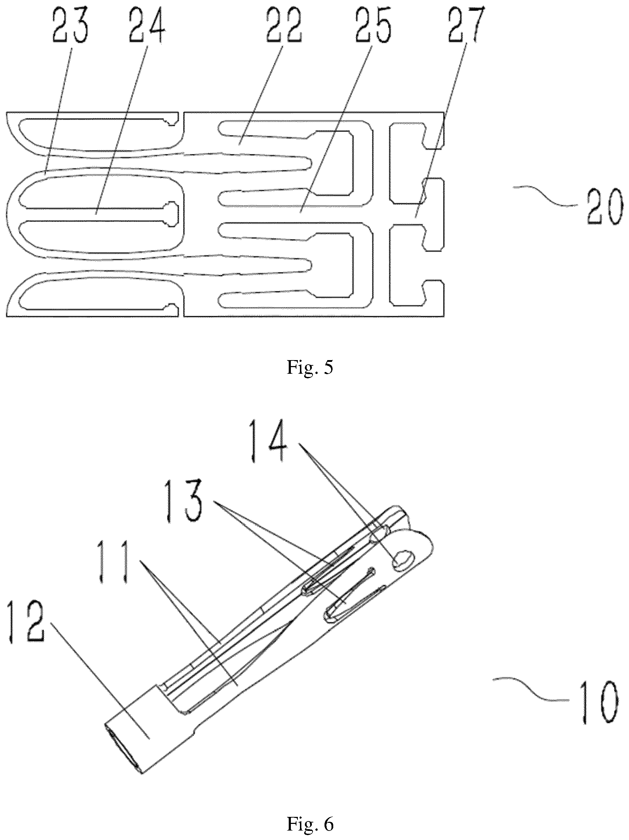

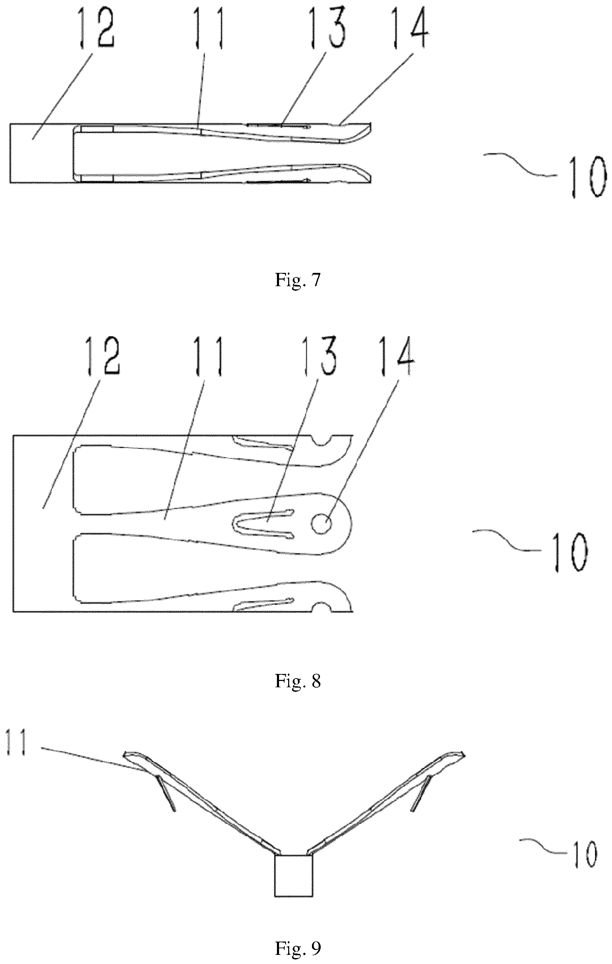

With reference to Figs. 6 to 9, the fixed arm mechanism 10 is formed by cutting a whole tube, which has good integrity and is easy to process. The fixed arm mechanism 10 also includes a fixed arm base 12, a barb 13 and a traction hole 14. Where, the fixed arm base 12 is located at the bottom part of the fixed arm mechanism 10, and the fixed arm base 12 is in a hollow cylindrical shape. Two fixed arms 11 are disposed symmetrically on the fixed arm base 12 and extend distally from the fixed arm base 12. Each fixed arm 11 is provided with at least one barb 13, which is protruding from the outer wall surface of the fixed arm 11. When the fixed arm 11 is in a folded state, the barb 13 tilts downward. The fixed arm 11 presses firmly against on the valve leaflet. The contact of the barb 13 and the valve leaflet may increase the friction force between the fixed arm 11 and the valve leaflet, thus enhancing the stability of clamping the valve leaflet. Each fixed arm 11 is provided with a traction hole 14 on the top part for the traction line passing through. The fixed arm base 12 is fixedly connected to the clamping arm base 21, e.g., the fixed arm base 12 is welded on the clamping arm base 21.

The fixed arm mechanism 10 and the clamping arm mechanism 20 are both made of shape memory alloy, e.g., NiTi, etc. The fixed arm 11 is deployed to a certain angle by pre-shaping. For example, the pre-shaped deployment angle of the fixed arm 11 is 120°, which is greater than the maximal deployment angle of the clamping arm 22, so as to ensure that the clamping arm 22 can still firmly press against on the clamping arm 22 when it deploys to the maximal angle to maintain the clamping effect over the valve leaflet. The surface of the fixed arm 11 and the clamping arm 22 are both covered with fiber cloth (not shown in the figure), e.g., PET cloth, which enables endothelial tissue to climb.

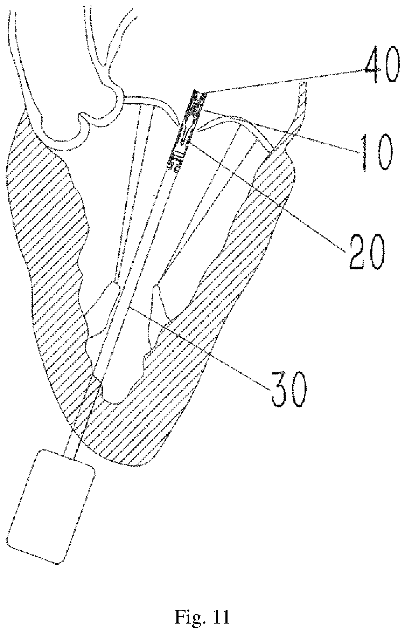

With reference to Fig. 11, the conveying system includes a conveying sheath tube 30, a push rod 40 and a traction line (not shown in the figure). The push rod 40 is slidably disposed inside the conveying sheath tube 30, provided with a fixed step, and is hollow inside. One end of the traction line is located outside the push rod, the other end is disposed inside the push rod 40 and protrudes out of the push rod 40, then passes through two traction holes 14 successively to return into the push rod 40 and protrude out of the push rod 40. The fixed step of the push rod 40 is abutted against the bottom surface of the fixed arm base 12, and the fixed arm 11 is in a folded state when the traction line is tightened. A T-shaped groove is provided on the top part of the conveying sheath tube 30, and the hanging head 27 of the clamp is clamped and fixed in the T-shaped groove.

The heart valve clamp provided in this embodiment is illustrated in detail with the treatment of mitral regurgitation as an example, in which the surgical pathway is a transcatheter approach to convey the conveying system to the location of the left atrium, and all subsequent operations are based on this.

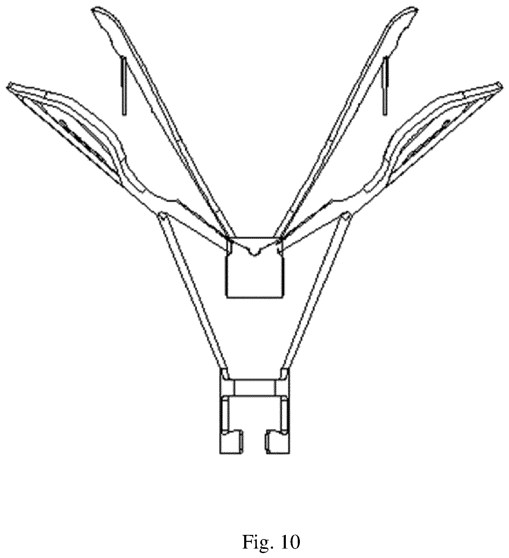

After placing the heart valve clamp at the location of the left atrium through the conveying system, the clamp is pushed to the lower part of the valve by the push rod 40, with the fixed step of the push rod 40 abutted against the bottom surface of the fixed arm base 12 to immobilize the fixed arm base 12 and the clamping arm base 21. The conveying sheath tube 30 is pulled downward, which drives the traction arm 25 to move downward so that the clamping arm 22 and the first supporting member 23 are deployed to a certain degree. The clamp is then pushed upward so that the clamping arm 22 and the first supporting member 23 hold the valve leaflet. The traction line is then released, and the fixed arm 11 expands and presses firmly on the valve leaflet and the clamping arm 22, with the state of the fixed arm 11 and the clamping arm 22 being both expanded as shown in Fig. 10. After confirming the clamping effect, the push rod 40 is folded, the conveying sheath tube 30 is rotated so that the hanging head 27 is disengaged from the T-shaped groove of the conveying sheath tube 30, completing the release of the heart valve clamp.

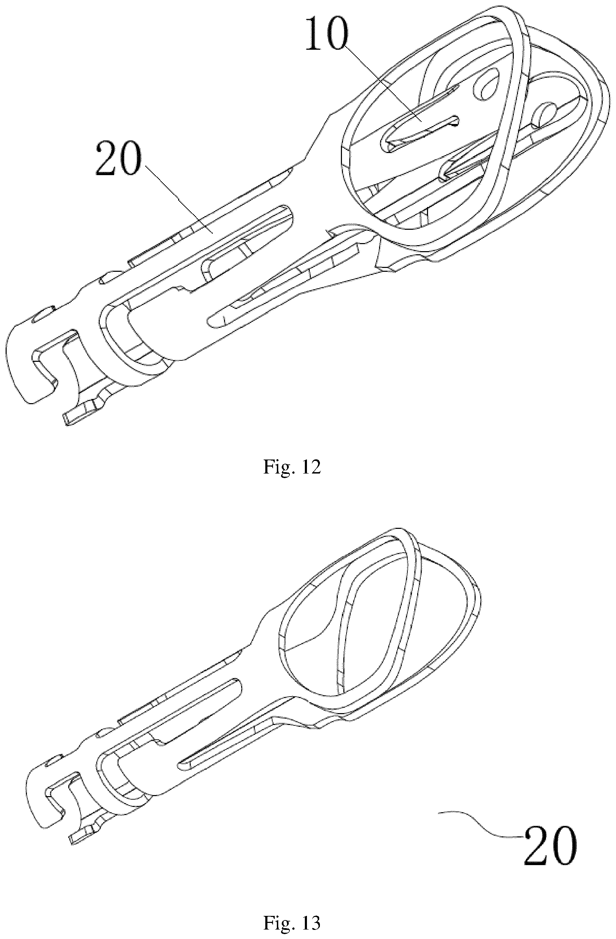

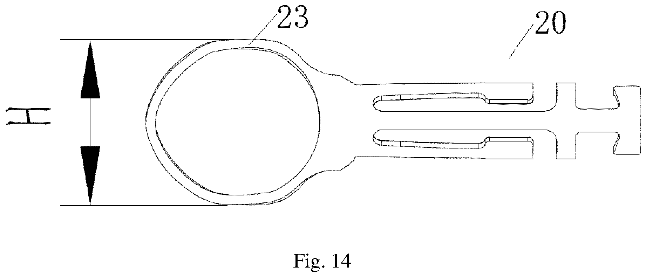

As an alternative implementation of Embodiment 1, with reference to Figs. 12 to 14, the second supporting member 24 may not be provided, then the first supporting member 23 is more flexible, with gentle contact with and less damage to the valve leaflet. As a modification, the first supporting member 23 may be shaped first and then welded on the clamping arm 22. The first supporting member 23 may also be any other shapes, such as rectangle, rhombus, etc. The first supporting member 23 may also be a solid structure, that is, not hollow in its central part, so long as that the width H of the first supporting member 23 is greater than the width of the clamping arm 22 to increase the clamping area of the clamping arm mechanism 20.

### Embodiment 2

A method for manufacturing a heart valve clamp is provided in this embodiment, which includes the steps below: processing to obtain an initial first supporting member 23 and a clamping arm 22; where a width of the clamping arm 22 is a first width; shaping the initial first supporting member 23 to obtain a first supporting member 23 having a width which is a second width, and connecting the first supporting member 23 to a distal end face of the clamping arm 22; where the second width is greater than the first width.

In such a method for manufacturing the heart valve clamp, an initial first supporting member 23 and a clamping arm 22 are first processed, the initial first supporting member 23 is then shaped to obtain a first supporting member 23 having a width greater than that of the clamping arm 22, which can effectively increase the clamping area of the clamping arm 22 and the first supporting member 23 when they clamp the valve leaflet. The clamping force of the clamp on the valve leaflet is uniform, which prevents the valve leaflet from being damaged, thus greatly reducing the operation risk during the implantation of the clamp. Moreover, after the initial first supporting member 23 is processed, it can be shaped according to the requirement of valves with different structures to obtain first supporting members 23 of different widths.

Further, in the step of processing to obtain the initial first supporting member 23 and the clamping arm 22, a tubular structure is cut to form the clamping arm 22 and the initial first supporting member 23 with a hollow central part that has a shape of a closed ring; the length of the initial first supporting member 23 in the extending direction of the clamping arm 22 is greater than the width of initial the first supporting member 23; in the step of shaping the initial first supporting member 23, a mold is placed in a hollow area of the initial first supporting member 23 to expand the initial first supporting member 23 for heat shaping.

The clamping arm 22 and the initial first supporting member 23 are formed by cutting a whole tubular structure, the processing is easy, the clamping arm mechanism 20 has a good integrity, the connections between the components are firm, and the clamping force is stable and reliable. The initial first supporting member 23 has a hollow central part that has a shape of a closed ring, which facilitates to expand it for heat shaping, and it is convenient to adjust the width of the first supporting member 23 to accommodate valves of different structures.

The length of the initial first supporting member 23 in the extending direction of the clamping arm 22 is greater than the width of the initial first supporting member 23. For example, an oval-shaped first supporting member 23 is cut on a tubular structure by laser cutting, which has rounded edges and corners, preventing the valve leaflet from being damaged when clamping. The length of the initial first supporting member 23 is smaller than its width, which facilitates expanding the initial first supporting member 23 distally in the direction of its width while shaping, to obtain first supporting members 23 of different widths. When expanded, the length of the initial first supporting member 23 becomes smaller, and its width becomes larger.

Obviously, the embodiments described above are merely examples for clear description and are not intended to limit the implementations. For those of ordinary skill in the art, other variations or changes in different forms can be made based on the above description. It is not necessary or possible to be exhaustive of all implementations here. Obvious variations or modifications derived therefrom are still within the protection scope of the present application.

## Claims

1. A heart valve clamp, comprising:
a fixed arm mechanism (10), comprising a fixed arm (11);
a clamping arm mechanism (20), comprising a clamping arm base (21), a clamping arm (22) and a first supporting member (23), wherein the clamping arm base (21) is connected to the fixed arm mechanism (10), the clamping arm (22) extends distally from the clamping arm base (21); a width of the clamping arm (22) is a first width;
wherein the first supporting member (23) is connected to a distal end face of the clamping arm (22) and extends distally in the extending direction of the clamping arm (22), a width of the first supporting member (23) is a second width, and the second width is greater than the first width.

2. The heart valve clamp according to claim 1, wherein, the first supporting member (23) is hollow in its central part to form a shape of a closed ring.

3. The heart valve clamp according to claim 2, wherein, before shaping, an initial length of the first supporting member (23) in the extending direction of the clamping arm (22) is greater than an initial width of the first supporting member (23).

4. The heart valve clamp according to claim 3, wherein, the first supporting member (23) is oval-shaped.

5. The heart valve clamp according to any one of claims 2-4, wherein, the fixed arm mechanism (10) is arranged inside the clamping arm mechanism (20), an outer wall surface of the fixed arm (11) is an arc-shaped surface;
a bottom part of the first supporting member (23) connected to the clamping arm (22) and a top part thereof opposite to the bottom part are both inclined outward, and the central part of the first supporting member (23) is concave inward; and when the clamp is in a folded state, the top part of the first supporting member (23) wraps around and fits over a distal end face of the fixed arm (11), and a central part of the fixed arm (11) protrudes into a hollow area of the first supporting member (23).

6. The heart valve clamp according to claim 3, wherein, a bottom part of the first supporting member (23) is provided with a first connecting member (231) extending toward the clamping arm (22).

7. The heart valve clamp according to any one of claims 1-6, wherein, the first supporting member (23) is integrally formed with the clamping arm (22).

8. The heart valve clamp according to any one of claims 2-6, wherein, the first supporting member (23) is provided with a second supporting member (24) extending in the extending direction of the clamping arm (22).

9. A method for manufacturing the heart valve clamp of any one of claims 1-8, comprising:
processing to obtain an initial first supporting member (23) and a clamping arm (22); wherein a width of the clamping arm (22) is a first width;
shaping the initial first supporting member (23) to obtain a first supporting member (23) having a width which is a second width, and connecting the first supporting member (23) to a distal end face of the clamping arm (22); wherein the second width is greater than the first width.

10. The method according to claim 9 for manufacturing the heart valve clamp, wherein,
in the step of processing to obtain the initial first supporting member (23) and the clamping arm (22), cutting a tubular structure to form the clamping arm (22) and the initial first supporting member (23) with a hollow central part that has a shape of a closed ring; an initial length of the first supporting member (23) in the extending direction of the clamping arm (22) is greater than an initial width of the first supporting member (23);
in the step of shaping the initial first supporting member (23), placing a mold in a hollow area of the initial first supporting member (23) to expand the initial first supporting member (23) for heat shaping.
